(19) Europäisches Patentamt
European Patent Office
Office européen des brevets

(11) EP 2 133 328 A2

(12) EUROPEAN PATENT APPLICATION

(43) Date of publication:
**16.12.2009 Bulletin 2009/51**

(51) Int Cl.:
*C07C 319/20* (2006.01) *C07C 319/28* (2006.01)
*C07C 323/58* (2006.01)

(21) Application number: **09162182.1**

(22) Date of filing: **08.06.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **09.06.2008 JP 2008150901**

(71) Applicant: **Sumitomo Chemical Company, Limited**
**Tokyo 104-8260 (JP)**

(72) Inventors:
- **Koizumi, Yoshiyuki**
  **Ehime 792-0025 (JP)**
- **Goto, Akinori**
  **Ehime 792-0009 (JP)**
- **Azemi, Takushi**
  **Ehime 792-0009 (JP)**

(74) Representative: **Vossius & Partner**
**Siebertstrasse 4**
**81675 München (DE)**

# (54) Process for producing methionine

(57) The present invention provides a process for producing methionine which is advantageous from the viewpoints of cost and wastewater disposal, and which comprises the following steps (1) to (5):
(1) a reaction step comprising hydrolysis of 5-[2-(methylthio)ethyl]imidazolidine-2,4-dione in the presence of a basic potassium compound;
(2) a first crystallization step comprising introduction of carbon dioxide into the reaction solution obtained in step (1) to precipitate methionine, and separation of the resultant slurry into a precipitate and a mother liquor;
(3) a second crystallization step comprising concentration of the mother liquor obtained in step (2), mixing with a lower alcohol, introduction of carbon dioxide into the mixture to precipitate methionine and potassium hydrogen carbonate, and separation of the resultant slurry into a precipitate and a mother liquor;
(4) a heating step comprising concentration of the mother liquor obtained in step (3) followed by a heating treatment at 150 to 200°C; and
(5) a third crystallization step comprising introduction of carbon dioxide into the heated mother liquor obtained in step (4) to precipitate methionine and potassium hydrogen carbonate, and separation of the resultant slurry into a precipitate and a mother liquor.



Printed by Jouve, 75001 PARIS (FR)

**Description**

BACKGROUND OF THE INVENTION

The present invention relates to a process for producing methionine by a hydrolysis reaction of 5-[(2-(methylthio) ethyl)]imidazolidine-2,4-dione [see the reaction scheme (1) shown below].

Reaction Scheme (1)

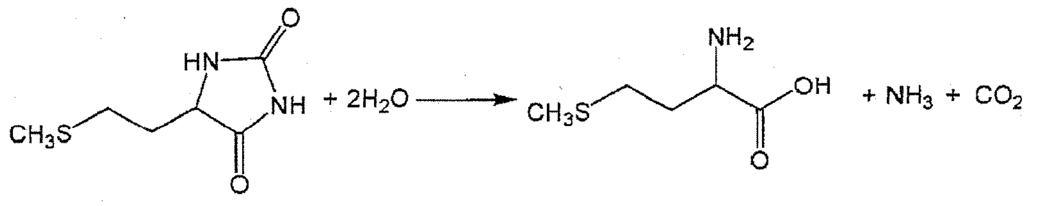

(1)

Methionine is useful as an additive for animal feed.

As an example of known processes for producing methionine, there is a process which comprises hydrolysis of 5-[(2-methylthio)ethyl]imidazolidine-2,4-dione under a basic condition using a basic potassium compound such as potassium carbonate or potassium hydrogen carbonate. According to this process, methionine can be separated and obtained as a crystal by introducing carbon dioxide into a reaction solution after hydrolysis to induce crystallization. However, the motherliquor after methionine separation still contains methionine in the amount corresponding to its solubility, and also contains potassium hydrogen carbonate, which can be recycled as the basic potassium compound. Therefore, the mother liquor may be recycled in the above hydrolysis reaction. However, the mother liquor must be purged at a predetermined rate because impurities are accumulated when the entire amount of the mother liquor is recycled. It is not preferable to dispose of the purged mother liquor as wastewater because methionine and potassium hydrogen carbonate contained therein are lost and there is also a great burden of wastewater disposal.

There have been reported various processes which comprise recovering methionine and potassium hydrogen carbonate as a so-called second crystal from the mother liquor. For example, JP-B 54-9174 discloses that the mother liquor is mixed with an aqueous solvent, for example, alcohol such as methyl alcohol, or acetone, and carbon dioxide is introduced into the mixture to induce crystallization. JP-A 51-1415 discloses that the mother liquor is concentrated and carbon dioxide is introduced into the concentrated solution to induce crystallization. JP-A 5-320124 discloses that the mother liquor is mixed with isopropyl alcohol and carbon dioxide is introduced into the mixed solution to induce crystallization. Furthermore, JP-A 2007-63141 discloses that the mother liquor after separation of a first crystal is concentrated, heated at 165°C and mixed with isopropyl alcohol, and then carbon dioxide is introduced into the mixture to induce crystallization. JP-A 2007-63141 also discloses a process in which a mother liquor after separation of a second crystal is concentrated and carbon dioxide is introduced into the concentrated solution, followed by crystallization to recover a third crystal.

However, according to the conventional processes as described above,when the motherliquor after separation of a second crystal is concentrated and crystallized, there is a problem of poor filterability for separating a third crystal, which resulted in high equipment costs.

SUMMARY OF THE INVENTION

An object of the present invention is to provide a process for producing methionine in which the recovery of a third crystal is efficiently carried out and thus which is also costwise advantageous.

The present inventors have
found that the amount of methionine dipeptide contained in a mother liquor after separation of a second crystal affects filterability of a third crystal, that is, when a large amount of methionine dipeptide is contained in the mother liquor, filterability of a third crystal after crystallization deteriorates. Based on this finding, the present inventors have found that filterability of a third crystal after crystallization is markedly improved by concentrating and then heating a mother liquor after separation of a second crystal to hydrolyze methionine dipeptide to methionine.

The present invention provides:

[1] A process for producing methionine, which comprises the following steps (1) to (5):

(1) a reaction step comprising hydrolysis of 5-[2-(methylthio)ethyl]imidazolidine-2,4-dione in the presence of a

basic potassium compound;
(2) a first crystallization step comprising introduction of carbon dioxide into the reaction solution obtained in step (1) to precipitate methionine, and separation of the resultant slurry into a precipitate and a mother liquor;
(3) a second crystallization step comprising concentration of the mother liquor obtained in step (2), mixing with a lower alcohol, introduction of carbon dioxide into the mixture to precipitate methionine and potassium hydrogen carbonate, and separation of the resultant slurry into a precipitate and a mother liquor;
(4) a heating step comprising concentration of the mother liquor obtained in step (3) followed by a heating treatment at 150 to 200°C; and
(5) a third crystallization step comprising introduction of carbon dioxide into the heated mother liquor obtained in step (4) to precipitate methionine and potassium hydrogen carbonate, and separation of the resultant slurry into a precipitate and a mother liquor;

[2] The process according to the above [1], wherein the lower alcohol used in the step (3) is selected from the group consisting of methyl alcohol, ethyl alcohol, n-propyl alcohol, isopropyl alcohol and t-butyl alcohol;
[3] The process according to the above [1] or [2], wherein the heating treatment of the step (4) is carried out for 0.3 to 10 hours; and
[4] The process according to any one of the above [1] to [3], wherein methionine and potassium hydrogen carbonate are precipitated in the presence of polyvinyl alcohol in the step (5).

According to the present invention, filterability upon separating a third crystal is improved, and therefore the recovery of the third crystal is carried out efficiently. As a result, methionine can be produced cost-effectively.

DETAILED DESCRIPTION OF THE PREFERRED EMBODIMENTS

In the present invention, 5-[(2-(methylthio)ethyl)]imidazolidine-2,4-dione as a starting material is hydrolyzed in the presence of a basic potassium compound to obtain a reaction solution containing methionine in the form of a potassium salt [the reaction step (1)]. The starting material 5-[(2-(methylthio)ethyl)]imidazolidine-2,4-dione can be produced by, for example, reacting 2-hydroxy-4-methylthiobutanenitrile with ammonia and carbon dioxide or with ammonium carbonate [see the following reaction scheme (2) or (3)].

Reaction Scheme (2)

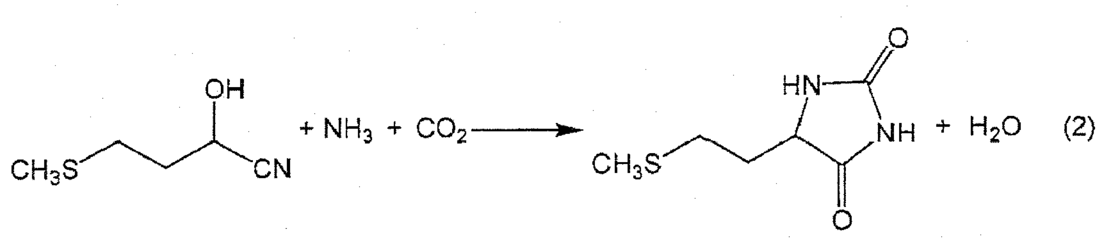

Reaction Scheme (3)

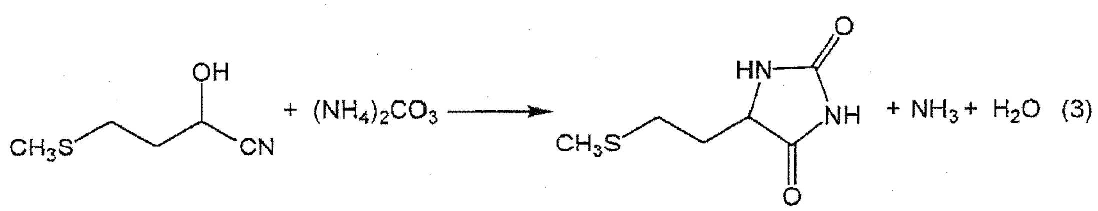

Examples of the basic potassium compound include potassium hydroxide, potassium carbonate and potassium hydrogen carbonate, and two or more of them can be used in combination, as needed. The used amount of the basic potassium compound is usually from 2 to 10 moles, preferably from 3 to 6 moles of potassium per 1 mol of 5-[(2-(methylthio) ethyl)]imidazolidine-2,4-dione. The used amount of water is usually from 2 to 20 times the weight of 5-[(2-(methylthio)ethyl)]imidazolidine-2,4-dione.

The hydrolysis reaction is preferably carried out under increased pressure at a gauge pressure of about 0.5 to 1 MPa while heating at about 150 to 200°C. The reaction time is usually 10 minutes to 24 hours.

In order to isolate methionine from the hydrolysis reaction solution thus obtained, carbon dioxide is introduced into the reaction solution to induce crystallization. The resultant slurry is separated into a precipitate and a mother liquor by filtration or decantation to obtain the precipitated methionine as a first crystal [the first crystallization step (2)].

As a result of introduction of carbon dioxide, carbon dioxide is absorbed into the reaction solution, and thus a potassium salt of methionine is precipitated as free methionine.

The introduction of carbon dioxide is preferably carried out under increased pressure at a gauge pressure of usually 0.1 to 1 MPa, preferably 0.2 to 0.5 MPa.

The crystallization temperature is usually 0 to 50°C, preferably 10 to 30°C. The crystallization time can be determined on the basis of the time until the hydrolysis reaction solution is saturated with carbon dioxide to sufficiently precipitate methionine, and it is usually 30 minutes to 24 hours.

Isolated methionine may be subjected to washing or pH adjustment as needed, and then dried to give a product. Drying is preferably carried out by heating at 50 to 120°C under slightly reduced pressure. The drying time is usually 10 minutes to 24 hours.

The mother liquor after separation of methionine (hereinafter, referred to as a "first crystal mother liquor") still contains methionine in the amount corresponding to its solubility, and also contains potassium hydrogen carbonate, which can be recycled as the basic potassium compound. Therefore, it is preferable to recycle the first crystal mother liquor in the hydrolysis reaction of step (1). However, the first crystal mother liquor also contains impurities present in starting materials and impurities derived from a side reaction upon hydrolysis, for example, amino acids other than methionine, such as glycine and alanine, and coloring components. Therefore, recycling of the first crystal mother liquor allows these impurities to be introduced into hydrolysis reaction. Thus, it is preferable to recycle the first crystal mother liquor in not the entire amount but in such an amount that impurities are not accumulated. The recycling amount of the first crystal mother liquor is usually 50 to 90% by weight, preferably 70 to 90% by weight of the entire amount of the first crystal mother liquor.

For recycling of the first crystal mother liquor, it is preferable that the first crystal mother liquor is concentrated and the concentrate is used as a recycling solution. Through the concentration step, carbon dioxide can be distilled off from the first crystal mother liquor, and thus a recycling solution which has increased basicity and is advantageous to hydrolysis reaction can be obtained. Furthermore, a conversion reaction of potassium hydrogen carbonate in the first crystal mother liquor into potassium carbonate ($2KHCO_3 \rightarrow K_2CO_3 + H_2O + CO_2$) is promoted by carrying out the concentration of the first crystal mother liquor at a high temperature of 100 to 140°C, and thus a recycling solution which has further increased basicity and is advantageous to hydrolysis reaction can be obtained. The concentration may be carried out under atmospheric pressure, reduced pressure or increased pressure. The concentration at high temperature as described above is carried out effectively under increased pressure. The concentration rate is usually from 1.2 to 4 times, preferably from 1.5 to 3.5 times. The "concentration rate" as used herein means the proportion of the weight of a solution before concentration to the weight of the solution after concentration (weight of solution before concentration/ weight of solution after conccntration).

The part of the first crystal mother liquor (concentrated first crystal mother liquor) which is not recycled is subjected to crystallization for the purpose of recovering methionine and potassium hydrogen carbonate as a second crystal. In the present invention, the second crystallization is accomplished by mixing the concentrated first crystal mother liquor with a lower alcohol and then introducing carbon dioxide into the mixed solution, and the resultant slurry is separated into a precipitate and a mother liquor by filtration or decantation to recover precipitated methionine and potassium hydrogen carbonate as a second crystal [the second crystallization step (3)]. It is also possible to subject the entire amount of the concentrated first crystal mother liquor to crystallization without recycling.

Examples of the lower alcohol include C1-C5 alkyl alcohols. Among them, preferred examples of the lower alcohol include alkyl alcohols miscible with water in a given ratio, such as methyl alcohol, ethyl alcohol, n-propyl alcohol, isopropyl alcohol and t-butyl alcohol, and particularly preferred is isopropyl alcohol. The used amount of the lower alcohol is usually 0.05 to 5 times, preferably 0.1 to 2 times the weight of the first crystal mother liquor subjected to crystallization. The first crystal mother liquor and the lower alcohol may be mixed before or simultaneously with introduction of carbon dioxide.

The first crystal mother liquor to be subjected to the second crystallization is concentrated before crystallization, similarly to the first crystal mother liquor to be recycled. Through the concentration step, the recovery rate of the second crystal can be increased. The concentration can be carried out under the same conditions as those for concentration of the first crystal mother liquor to be recycled. After the entire amount of the first crystal mother liquor is concentrated, the concentrate may be separated into two parts respectively for recycling and for second crystallization.

In the concentration step of the first crystal mother liquor, the basicity of the mother liquor increases and thereby the free methionine obtained by the first crystallization step returns to a potassium salt of methionine. Thus, also in the

second crystallization step, carbon dioxide is introduced into the mixed solution of the concentrated first crystal mother liquor and the lower alcohol to convert a potassium salt of methionine into free methionine again.

The first crystal mother liquor is preferably heated after concentration to hydrolyze methionine dipeptide (dehydrated condensate of two methionine molecules) contained therein, and thereby regeneration of methionine is promoted. The heating treatment is preferably carried out under increased pressure at a gauge pressure of about 0.5 to 2 MPa at around 140 to 180°C. The heating treatment time is usually 10 minutes to 24 hours.

The introduction of carbon dioxide is carried out under increased pressure at a gauge pressure of usually 0.1 to 1 MPa, preferably 0.2 to 0.5 MPa, similarly to the first crystallization step.

The crystallization temperature is usually 0 to 50°C, preferably 5 to 20°C. The crystallization time may be determined on the basis of the time until the mixed solution is saturated with carbon dioxide to sufficiently precipitate methionine and potassium hydrogen carbonate, and is usually 10 minutes to 24 hours.

It is preferable that the second crystal (mixture of methionine and potassium hydrogen carbonate) thus recovered is recycled in the hydrolysis reaction of the step (1). In this case, it is preferable in view of operability that the recovered second crystal is dissolved in the first crystal mother liquor for recycling and then subjected to recycling.

A mother liquor after separation of the second crystal (hereinafter, referred to as a "second crystal mother liquor") still contains methionine and potassium hydrogen carbonate. Thus, in the present invention, methionine and potassium hydrogen carbonate are recovered as a third crystal from the second crystal mother liquor by the following procedure. The second crystal mother liquor is concentrated and then subjected to a heating treatment [the heating step (4)]. Then, carbon dioxide is introduced into the solution obtained in the heating step (4) to induce crystallization, and the resultant slurry is separated into a precipitate and a mother liquor by filtration or decantation to recover methionine and potassium hydrogen carbonate as a third crystal [the third crystallization step (5)].

Through the concentration of the second crystal mother liquor, a recovery rate of the third crystal can be increased. The concentration can be carried out under the same conditions as those for concentration of the first crystal mother liquor to be recycled.

The heating treatment of the second crystal mother liquor after concentration leads to hydrolysis of methionine dipeptide contained therein, and thereby regeneration of methionine is promoted. The heating treatment is carried out under increased pressure such as a gauge pressure of about 0.5 to 2 MPa and at around 150 to 200°C, preferably 160 to 180°C. The heating treatment time is preferably 0.3 to 10 hours, more preferably 0.5 to 5 hours.

It is preferable that the heating treatment is carried out until the proportion of methionine dipeptide to methionine reaches preferably 5 to 50% by weight, more preferably 5 to 30% by weight.

Heretofore, after concentration of a second crystal mother liquor, carbon dioxide was directly introduced into the concentrate which had not been heated to induce crystallization, and the resultant slurry was separated into a precipitate and a mother liquor to obtain a third crystal (e.g., see JP-A 2007-63141). However, according to this conventional process, filterability of the third crystal was very poor upon separation of the third crystal. The present inventors have intensively studied to clear the reason thereof. As a result, the present inventors have found that the amount of methionine dipeptide present in a second crystal mother liquor affects the filterability of a third crystal.

The present inventors also have found that filterability of a second crystal is good even if carbon dioxide is directly introduced into a concentrate of a first crystal mother liquor which is not heated, to induce crystallization. The reason is probably because the content of methionine dipeptide in the second crystal mother liquor is higher than that in the first crystal mother liquor and the content of methionine dipeptide in the first crystal mother liquor does not affect the filterability of the second crystal.

Also in the concentration step of the second crystal mother liquor, the basicity of the mother liquor increases and thereby the free methionine obtained by the second crystallization step returns to a potassium salt of methionine. Thus, also in the third crystallization step, carbon dioxide is introduced into the concentrated and heated second crystal mother liquor to convert a potassium salt of methionine into free methionine again.

The introduction of carbon dioxide is carried out under increased pressure at a gauge pressure of usually 0.1 to 1 MPa, preferably 0.2 to 0.5 MPa, similarly to the first crystallization step and the second crystallization step.

The crystallization temperature is usually 0 to 50°C, preferably 5 to 30°C. The crystallization time may be determined on the basis of the time until the concentrated and heated second crystal mother liquor is saturated with carbon dioxide to sufficiently precipitate methionine and potassium hydrogen carbonate, and is usually 10 minutes to 24 hours.

The third crystallization step is preferably carried out in the presence of polyvinyl alcohol, for example, as shown in JP-A 4-169570. The presence of polyvinyl alcohol upon crystallization allows the third crystal to be precipitated in a good deliquoring form and thereby, upon the subsequent solid-liquid separation,the mother liquor hardly remains in the third crystal. As a result, the content of impurities in the recovered third crystal can be reduced. The used amount of polyvinyl alcohol is usually 100 to 5,000 ppm by weight, preferably 200 to 3,000 ppm by weight in the second crystal mother liquor after the heating step (4).

The first and second crystallization steps can also be carried out in the presence of polyvinyl alcohol. It is

particularly preferable that the first crystallization step is carried out in the presence of polyvinyl alcohol because methionine having a good product powder property can be obtained.

The third crystal (mixture of methionine and potassium hydrogen carbonate) thus recovered is preferably recycled in the hydrolysis reaction of the step (1), similarly to the second crystal.

All of the steps (1) to (5) may be carried out continuously or batch-wise. Alternatively, some of the steps (1) to (5) may be carried out continuously and some of them may be carried out batch-wise.

EXAMPLES

Hereinafter, the present invention will be explained by means of Examples. The present invention is not limited thereto. In Examples, the terms "%" and "part(s)" means concentration or a used amount based on weight, unless otherwise specified.

Example 1

Reaction Step (1)

While 100 parts per hour of a 18.7% solution of 5-[2-(methylthio)ethyl]imidazolidine-2,4-dione in water, 1.0 part per hour of potassium hydroxide, 67.6 parts per hour of a previously prepared first concentrated solution of a first crystal mother liquor (containing 6.0% of methionine and 13.5% of potassium), 25.8 parts per hour of a previously prepared second crystal-containing solution (containing 7.6% of methionine and 18.2% of potassium, and prepared by dissolving a wet cake of the second crystal in a concentrate of the first crystal mother liquor and then concentrating the solution) were charged into a reactor, hydrolysis reaction was carried out under a gauge pressure of 0.88 MPa at 173 to 178°C for 1 hour of residence time.

First Crystallization Step (2)

The reaction solution obtained by the above hydrolysis reaction (133.1 parts per hour), 60.7 parts per hour of water and 0.023 parts per hour of polyvinyl alcohol were mixed, charged into a crystallizer, and subjected to crystallization under increased pressure of carbon dioxide at a gauge pressure of 0.3 MPa at 20°C to precipitate methionine. The resultant slurry was filtered. The residue was washed with water and then dried under slightly reduced pressure at 85 to 105°C to obtain 15.6 parts per hour of methionine (99.6% purity, 97% yield). At the same time, 184.0 parts per hour of a first crystal mother liquor was recovered as the filtrate.

The first crystal mother liquor (184.0 parts per hour) was charged into a concentrator and concentrated under a gauge pressure of 0.2 MPa at 115°C and then at 140°C to obtain 106.4 parts per hour of a first concentrate (first concentration rate: 1.7 times). As a result of analysis, the first concentrate contained 6.0% of methionine and 13.5% of potassium, and the proportion of methionine dipeptide to methionine in the first concentrate was 36.5%.

A part (67.6 parts per hour) of the first concentrate of the first crystal mother liquor (106.4 parts per hour) was recycled to the hydrolysis reaction, as described above. Further, 18.5 parts per hour of the first concentrate was charged into a heater, subjected to a heating treatment under a gauge pressure of 1 MPa at 165°C for 1 hour of residence time, charged into a concentrator, and then concentrated under a gauge pressure of 0.2 MPa at 135°C to obtain 12.3 parts per hour of a second concentrate (second concentration rate: 1.5 times; total concentration rate of first and second concentration: 2.6 times). Further, the remaining 20.3 parts per hour of the first concentrate was used for dissolving a wet cake of a second crystal as described below.

Second Crystallization Step (3)

The second concentrate of the first crystal mother liquor (12.3 parts per hour) and 3.3 parts per hour of isopropyl alcohol were mixed, charged into a crystallizer, and subjected to crystallization under increased pressure of carbon dioxide at a gauge pressure of 0.3 MPa at 12 to 16°C. The resultant slurry was filtered to obtain 7.8 parts per hour of a wet cake of a second crystal as a residue At the same time, 9.1 parts per hour of a second crystal mother liquor were recovered as a filtrate.

The second crystal wet cake (7.8 parts per hour) was dissolved in the remaining first concentrate of the first crystal mother liquor (20.3 parts per hour) as described above, charged into a concentrator and then concentrated under atmospheric pressure at 80°C to distill off isopropyl alcohol contained in the second crystal and obtain 25.8 parts per hour of a solution of the second crystal. As a result of analysis, the second crystal solution contained 7.6% of methionine and 18.2% of potassium. The second crystal solution (25.8 parts per hour) was recycled to the hydrolysis reaction, as described above.

Heating Step (4) and Third Crystallization Step (5)

The second crystal mother liquor (9.1 parts per hour) was charged into a concentrator and concentrated under atmospheric pressure at 80 to 110°C to distill off isopropyl alcohol and obtain 6.0 parts per hour of a first concentrate (first concentration rate: 1.5 times). As a result of analysis, the first concentrate contained 3.14% of methionine and 7.25% of potassium, and the proportion of methionine dipeptide to methionine in the first concentrate was 102.9%.

A part of the first concentrate of the second crystal mother liquor was charged into a concentrator and then concentrated under reduced pressure at an absolute pressure of 60 mmHg (8 kPa) at 60°C until a second concentration rate reached 2.3 times (total concentration rate of first and second concentration: 3.5 times). As a resuit of analysis, the second concentrate contained 0.690 of glycine and 1.07% of alanine.

The second concentrate above was put in a heater and heated at 180°C for 4 hours. As a result of analysis, the second concentrate after heating contained 10.79% of methionine, 1.93% of methionine dipeptide and 12.2% of potassium, and the proportion of methionine dipeptide to methionine in the heated second concentrate was 17.9%.

The heated second concentrate above was put in a crystallizer and subjected to crystallization under increased pressure of carbon dioxide at a gauge pressure of 0.3 MPa at 15°C after adding 500 ppm by weight of polyvinyl alcohol to precipitate methionine and potassium hydrogen carbonate. The resultant slurry was filtered under increased pressure at a filtration gauge pressure of 0.3 MPa. At this point, the specific resistance to filtration of a wet cake was 0.64 x $10^{10}$ m/kg as measured according to a procedure described below.

Comparative Example 1

The second concentrate of the second crystal mother liquor obtained in Example 1 was put in a crystallizer and subjected to crystallization under increased pressure of carbon dioxide at a gauge pressure of 0.3 MPa at 15°C to precipitate methionine and potassium hydrogen carbonate. The resultant slurry was filtered under increased pressure at a filtration gauge pressure of 0.3 MPa. At this point, the specific resistance to filtration of a wet cake was 2.0 x $10^{10}$ m/kg as measured according to a procedure described below.

Reference Example 1

The first concentrate of the first crystal mother liquor obtained in Example 1 (18.5 parts per hour) was concentrated under increased pressure at a gauge pressure of 0.2 MPa at 135°C to obtain 12.3 parts per hour of a second concentrate (second concentration rate: 1.5 times, total concentration rate of first and second concentration: 2.6 times). The second concentrate (12.3 parts per hour) and 3.3 parts per hour of isopropyl alcohol were mixed and charged into a crystallizer, and subjected to crystallization under increased pressure of carbon dioxide at a gauge pressure of 0.3 MPa at 12 to 16°C. The resultant slurry was filtered under increased pressure at a filtration gauge pressure of 0.3 MPa. At this point, the specific resistance to filtration of a wet cake was 0.55 x $10^{10}$ m/kg as measured according to a procedure described below.

Measurement of Specific Resistance to Filtration

A filtration rate was measured by the following procedure, and a specific resistance to filtration was calculated.

Slurry was put in a pressure-resistant vessel. The vessel was sealed and pressurized to a predetermined pressure. A vent valve at the bottom was opened, and filtration was started. The weight of the filtrate was measured at every predetermined time,and the filtration rate was calculated. At the point when the filtrate no longer dropped, the pressure was released and the vessel was opened. The thickness of the wet cake was measured, and it was taken out of the vessel. The weight and water content of the wet cake were measured (the water content was measured by Karl Fischer method).

Based on data of a filtration rate, a filtration area, the viscosity of a filtrate, the weight, water content and thickness of a wet cake, and a filtration pressure, a specific resistance to filtration was calculated according to the following equation:

$$\theta/V = V/K + 2V_0/K$$

wherein,

V: Cumulative volume of a filtrate

$K = 2 \cdot \Delta P \cdot A^2 \cdot gc/(\mu \cdot \alpha m \cdot C)$
$\mu$: Viscosity of a filtrate
$V_0 = Rm \cdot A/(\alpha m \cdot C)$
$\theta/V$: Reciprocal number of filtration rate
$\Delta P$: Pressure difference (= filtration pressure)
A: Filtration area
gc: Gravitational acceleration
$\alpha m$: Specific resistance to filtration
C: Solid concentration
Rm: Filter cloth resistance.

As for the measurements of $\alpha m$ and Rm, Kagaku-kogaku-binran, pp.803-804, edited by the Society of Chemical Engineers, Japan, published by Maruzen, 1999 can be referenced.

According to the present invention, filterability is improved upon separation of a third crystal, and therefore the third crystal is recovered efficiently. As a result, methionine can be produced cost-effectively.

## Claims

**1.** A process for producing methionine, which comprises the following steps (1) to (5):

(1) a reaction step comprising hydrolysis of 5-[2-(methylthio)ethyl]imidazolidine-2,4-dione in the presence of a basic potassium compound;
(2) a first crystallization step comprising introduction of carbon dioxide into the reaction solution obtained in step (1) to precipitate methionine, and separation of the resultant slurry into a precipitate and a mother liquor;
(3) a second crystallization step comprising concentration of the mother liquor obtained in step (2), mixing with a lower alcohol, introduction of carbon dioxide into the mixture to precipitate methionine and potassium hydrogen carbonate, and separation of the resultant slurry into a precipitate and a mother liquor;
(4) a heating step comprising concentration of the mother liquor obtained in step (3) followed by a heating treatment at 150 to 200°C; and
(5) a third crystallization step comprising introduction of carbon dioxide into the heated mother liquor obtained in step (4) to precipitate methionine and potassium hydrogen carbonate, and separation of the resultant slurry into a precipitate and a mother liquor.

**2.** The process according to claim 1, wherein the lower alcohol used in step (3) is selected from the group consisting of methyl alcohol, ethyl alcohol, n-propyl alcohol, isopropyl alcohol and t-butyl alcohol.

**3.** The process according to claim 1 or 2, wherein the heating treatment of step (4) is carried out for 0.3 to 10 hours.

**4.** The process according to any one of claims 1 to 3, wherein methionine and potassium hydrogen carbonate are precipitated in the presence of polyvinyl alcohol in step (5).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 54009174 B
- JP 51001415 A
- JP 5320124 A
- JP 2007063141 A
- JP 4169570 A


### Non-patent literature cited in the description


- Kagaku-kogaku-binran. Maruzen, 1999, 803-804